# EUROPEAN PATENT APPLICATION

(11) **EP 0 534 360 A1**
(43) Date of publication of application: **31.03.1993**
(21) Application number: 92116176.6
(22) Date of filing: 22.09.1992
(51) Int. Cl.: C07C 309/40, C07C 303/06

(54) **Method of preparing nitrotoluenesulfonic acids and sodium salts thereof**

(30) Priority: 26.09.1991 JP 247460/91; 14.01.1992 JP 4962/92
(71) Applicant: NISSAN CHEMICAL INDUSTRIES LTD., Chiyoda-ku Tokyo (JP)
(72) Inventor: Suzuki, Fumio, c/o Nissan Chemical Ind., Ltd., Funabashi-shi, Chiba-ken (JP); Asamidori,Yasunobu, c/o Nissan Chemical Ind., Ltd., Funabashi-shi, Chiba-ken (JP); Nakai, Tadao, c/o Nissan Chemical Ind., Ltd., Nagoya-shi, Aichi-ken (JP); Kimura, Hiroshi, c/o Nissan Chemical Ind., Ltd., Nagoya-shi, Aichi-ken (JP); Kubo, Masao, c/o Nissan Chemical Ind., Ltd., Chiyoda-ku, Tokyo (JP)
(74) Representative: Hansen, Bernd, Dr. Dipl.-Chem.

(57) **Abstract**

Method of sulfonating a nitrotoluene to produce a toluenesulfonic acid and its sodium salt, which method is free from a waste acid being generated and is free from a non-reacted nitrotoluene being retained in the product.

## Description

### BACKGROUND OF THE INVENTION

### 1. Field of the Invention

The present invention relates to methods of preparing 2-methyl-5-nitrobenzenesulfonic acid (hereinafter referred to as "PNT-sulfonic acid") and sodium 2-methyl-5-nitrobenzenesulfonate (hereinafter referred to as sodium PNT-sulfonate"), which are used as intermediates of preparing optical brighteners, dyes, pigments and others, and also to methods of preparing 4-methyl-3-nitrobenzenesulfonic acid (hereinafter referred to as "ONT-sulfonic acid") and sodium 4-methyl-3-nitrobenzenesulfonate (hereinafter referred to as "sodium ONT-sulfonate"), which are used as intermediates of similarly preparing dyes and others.

### 2. Description of the Prior Art

Heretofore, various methods have been investigated for sulfonation of p-nitrotoluene (hereinafter referred to as "PNT"). For instance, in the case of a mehtod of using a fuming sulfuric acid, it is generally conducted at an SO₃ concentration of from 20 to 25 wt.%. However, since it is accompanied by a large amount of a waste acid, sulfonation is effected with from 50 to 85 wt.% of a fuming sulfuric acid and, after dilution with water, PNT-sulfonic acid is obtained by crystallization at a sulfuric acid concentration of from 60 to 75 wt.% (Japanese Patent Application Laid-Open No. Sho 60-132950 (132950/1985)).

However, the method merely reduces a small amount of the waste acid and still involves a problem on the treatment of the waste acid.

In accordance with a method of using 100 % SO₃ gas for sulfonation, a small amount of sulfuric acid (0.14 part per 1.0 part of PNT) is used so as to promote the reaction and to improve the fluidity of the reaction liquid at the end of the reaction (Japanese Patent Application Laid-Open No. Sho 58-118555 (118555/1983)). Where SO₃ gas is used, sulfonation reaction becomes suddenly slow when the sulfonation yield has reached from 60 to 70 %. (Fine Aromatics Intermediates, written by N. N. WOROSHZOW, published by Gihodo Publishing Co., page 48). Therefore, it is necessary to use sulfuric acid or excess SO₃ for promotion and completion of the reaction. Anyhow, after the reaction product has been dissolved in water, a fairly large amount of sulfuric acid would remain in PNT-sulfonic acid (Example 1 of the above-mentioned Japanese Patent Application Laid-Open No. Sho 58-118555; H₂SO₄/PNT-sulfonic acid = 4.5 %/33.6 %), and the amount of the sulfuric acid would often be a bar to the next step where the product is used directly therein, e.g., further purification is necessary for removing the remaining sulfuric acid and production of a waste sulfuric acid as a by-product is inevitable. The above is applied to the case of unreated ONT.

20 vol. % SO₃ gas (inert gas nitrogen, 80 vol.%) induces a milder reaction than the case of 100 % SO₃ gas, but completion of the reaction with it is difficult so that the unreacted PNT and coloring components are removed by adsorption with an active charcoal (U.S. Patent No. 3,840,591). In this case, however, a fairly large amount of an active charcoal is necessary, which is expensive.

A method is known, in which 100 % SO₃ gas is used to obtain a product with conversion of 70 % or more, the product is dissolved in water and the unreacted PNT is extracted out therefrom with a water-immiscible solvent (Japanese Patent Publication No. Sho 63-40184 (40184/1988)). However, it involves troublesome operations of recovery of the used solvent and removal of the remaining solvent.

On the other hand, for sulfonation of o-nitrotoluene (hereinafter referred to as "ONT"), there are known a method of using a fuming sulfuric acid (for example, Journal of Japan Chemical Society, 1977 (11), pp. 1694 to 1697) and a method of using a liquid SO₃ (for example, British Patent No. 2,170,195).

In accordance with the sulfonation with a fuming sulfuric acid, somewhat excess fuming sulfuric acid is used to effect sulfonation, the reaction product from which the unreacted ONT has disappeared is added with an aqueous saline solution, or is diluted with water and then is added with a salt to form a sodium sulfonate, which is obtained by salting-out (Fine Aromatics Intermediates, written by N. N. WOROSHZOW, published by Gihodo Publising Co., page 55).

However, this method generates a large amount of a waste acid containing organic substances and therefore involves a problem on the treatment of it.

### SUMMARY OF THE INVENTION

Thus, the present invention is to provide a method in which, in preparation of nitrotoluenesulfonic acids and sodium nitrotoluene-sulfonates by sulfonating nitrotoluenes with an SO₃ gas, products having a reduced amount of remaining sulfuric acid (or Gluaber's salt for sodium salts) and being free from an unreacted nitrotoluene are obtained.

Another object of the present invention is to provide a method in which a nitrotoluene is sulfonated with SO₃ gas, whereupon SO₃ is used in an amount of from 90 mol% to 130 mol% of the necessary theoretical amount for sulfonation, then the sulfonation reaction mixture is ripened so as to reduce the unreacted nitrotoluene and SO₃ due to interaction of the remaining nitrotoluene and SO₃, and thereafter the unreacted nitrotoluene is removed to obtain a nitrotoluenesulfonic acid and its sodium salt which are directly used as intermediates for preparing optical brighteners, dyes, pigments and others.

### DESCRIPTION OF THE PREFERRED EMBODIMENTS

The present invention will be explained in order of the steps hereunder.

Sulfonation of nitrotoluene with SO₃ gas may be effected by a continuous process or a batch-wise process, at a temperature of from the melting point to 130 °C. In order to reduce the amount of the remaining unreacted nitrotoluene, the method of the present invention needs a ripening step of maintaining the reaction system at a temperature of from 100 °C to 130 °C without introduction of SO₃ gas thereinto, after the previous sulfonation step of introducing SO₃ gas into the reaction system. If the temperature is higher than 130 °C, the color of the obtained sulfonic acid product would be extremely bad. If desired, a known sulfone inhibitor of inhibiting formation of dimethyl-dinitrobiphenylsulfones (hereinafter referred to as "sulfones") may be used in carrying out the method of the invention. SO₃ is used at the concentrations of 3 to 50 vol.%, preferably, 5 to 40 vol.%. In a case of the concetration being less than 3 vol/%, it is not preferable because the reaction efficiency is worse. On the other hand, in a case of the concentration being more than 50 vol.%, it is not preferable because a reaction product is colored.

In bacth-wise sulfonation of a nitrotoluene with an SO₃ gas, a half of the step is effected at a relatively low temperature within the scope of the above-mentioned reaction temperature range, for example, the case of PNT being at from 60 °C to 105 °C and the case of ONT being at from room temperature to 85 °C, so that escape of the starting material nitrotoluene from the reaction system is prevented. This is especially effective, when the SO₃ concentration in the reaction material gas is low due to the inert gas (dry air or nitrogen) therein. In the latter half of the reaction step, escape of the starting material is little and the reaction speed lowers, so that the reaction temperature is elevated up to from 105 °C to 115 °C.

The amount of SO₃ gas to be charged to the reaction system is from 90 to 130 mol% of the theoretical amount necessary for sulfonation. Preferably, it is from 95 to 120 mol% in a case that SO₃ containing an inert gas is used. Where SO₃ is diluted more with an inert gas therein, the reaction with it would be milder. On the contrary, however, absorption of SO₃ by the reaction system would be worse in the case, so that a large amount of SO₃ is necessary. In the latter half of the sulfonation step, even if the necessary amount of SO₃ is applied to the system at a reaction temperature of from 105 °C to 115 °C, several % or more nitrotoluenes would remain. It may be presumed that they would remain as pyrosulfonic acids to be formed by adding SO₃ to nitrotoluenesulfonic acids and as dissolved SO₃. In order to accelerate the reaction between the remaining nitrotoluenes and SO₃, ripening of the reaction system at 105 °C to 115 °C for further about 3 hours is effected to give nitrotoluenesulfonic acids. The amount of SO₃ to be initially used is so controlled that the amount of the still remaining nitrotoluenes could be about 0.5 % or more after ripening, whereby the content of the sulfuric acid after dissolved in water may be reduced.

The reaction product after ripened is dissolved in water to give an aqueous solution thereof, which is then heated so that nitrotoluenes are distilled out along with water and thus, the unreacted nitrotoluene are removed. In the step, where the concentration of the resulting solution is needed to be kept constant, water of the same amount as the distilling-out water is supplemented to the distillation system. Steam distillation of blowing steam into the distillation system may also be employed. In order to remove the sulfones to be produced as by-products, the aqueous solution is made to have a concentration of about 50 wt.% or less, preferably from 30 to 45 wt.%, and is cooled so that the sulfones precipitated out are separated and removed. If the concentration is more than 50 wt.%, dissolution of sulfones would increase disadvantageously. The step of removing sulfones may be effected either before or after removal of the unreacted nitrotoluenes.

Where an aqueous solution of a nitrotoluenesulfonic acid is desired to be obtained as a final product, removal of sulfones is preferably effected after removal of the unreacted nitrotoluenes in view of effective utilization of the heat as generated in dissolution of the product in water. On the other hand, where unreacted nitrotoluenes are removed by steam distillation using a packed column, removal of sulfones before the steam distillation is preferable. Where a sodium nitrotoluene-sulfonate is desired to be obtained as a final product, removal of sulfones before neutralization (i.e., before the removal of unreacted nitrotoluenes) is preferred since the solubility of the sodium salt is low.

In this case, the reaction product after ripened is dissolved in water to give an aqueous 30 to 45 wt.% solution and cooled, and the sulfones as precipitated out are removed by filtration. Afterwards, it is then neutralized with, for example, an aqueous 30 % sodium hydroxide solution to give an aqueous solution of a sodium nitrotoluene-sulfonate. This is then further heated so as to remove nitrotoluenes by distillation along with water, whereby the unreacted nitrotoluenes are removed. In this case, water of the same amount as the amount of the distilled water may be supplemented so as to maintain the concentration of the solution, or a steam distillation method of blowing steam into the reaction system may be employed. Where the aqeuous solution of a sodium nitrotoluene-sulfonate, from which unreacted nitrotoluenes have been removed, is needed to be further concentrated, the amount of water for dissolution as well as the amount of water to be supplied during distillation may be reduced to attain the desired concentration. The concentration for easy operation in this case is, for example, from 25 to 30 wt.% as sodium PNT-sulfonate or from 30 to 40 wt.% as sodium ONT-sulfonate.

The thus obtained thick solution may be strongly stirred with a kneader or the like and concentrated to give a thick slurry, or it may be dried with a drying machine such as a drum drier to give a solid powder.

An aqueous solution of a nitrotoluenesulfonic acid as formed by dissolving the ripened reaction product in water, from which sulfones as precipitated out after cooling have been removed, or an aqueous solution of the same which has further been neutralized, i.e., an aqueous solution of sodium nitrotoluene-sulfonate, is charged from the top of a packed column, while a steam is blown thereinto from the bottom of the column as a countercurrent stream, whereby the unreacted nitrotoluenes may be removed from the solution. The amount of the steam to be used in the continuous process is fairly smaller than that in the batch-wise process. If a further concentrated aqueous solution of a nitrotoluenesulfonic acid or an aqueous solution of a sodium nitrotoluene-sulfonate is desired to be obtained, a distillation tube which is equipped at the bottom of the packed column and this is heated so that the steam to be generated by heating along with concentration may be removed by steam distillation removal. This is an effective method of removing nitrotoluenes from the product solution.

### EXAMPLES:

The present invention will be explained in more detail by way of the following examples, which, however, are not intended to restrict the scope of the present invention.

### EXAMPLE 1

4.87 kg (35.5 mols) of a starting material of a molten PNT was charged into a 10 liter-reactor (made of stainless steel; SUS304) equipped with a stirrer, a baffle and a gas-inlet tube. 1.6 m³/h of a dry air was introduced thereinto from the gas-inlet tube for 5 minutes so that PNT in the tube was purged out. Next, 1.6 m³/h of 9 vol.% SO₃ gas was introduced from the tube to initiate sulfonation. The temperature was gradually elevated from 60 °C up to 105 °c over a period of about 2 hours. Further, SO₃ gas (108 mol% of PNT) was introduced at from 105 °C to 115 °C for 4 hours, and the reaction was stopped.

The unreacted PNT at the point was 9.0 %, as measured by HPLC (high performance liquid chromatography) analysis. Further, the reaction system was ripened for 3 hours at the same temperature, whereby the amount of the unreacted PNT decreased to 3.6 %. For analysis, the product was hydrolyzed, and the sulfuric acid formed was analyzed by potentiometric titration analysis to be 3.6 wt.%. The purity of the thus obtained PNT-sulfonic acid was 93.3 wt.%; and the yield thereof was 95 %.

500 g of the ripened product (PNT content: 3.6 wt.%) was dissolved in 600 g of water and heated at 80 °C for 30 minutes to give an aqueous solution of a crude PNT-sulfonic acid. The solution was heated further so as to distill out PNT along with water, while water of the same amount as that of the distilled water was continuously supplemented to the system. The distilled water was cloudly as containing PNT as suspended therein. After 1600 ml of water was distilled out, the distilled water became transparent. Further, 200 ml of water was distilled out, whereupon the unreacted PNT could no more be detected in the aqeuous solution of PNT-sulfonic acid in the reactor. The solution was cooled and 4.5 g of PNT-sulfone as precipitated out was removed by filtration. Thus, 1100 g of 42.4 % aqeuous solution of PNT-sulfonic acid was obtained.

### EXAMPLE 2

500 g of the same ripened reaction product as that in EXAMPLE 1 (PNT content: 3.6 wt.%) was dissolved in water, and the resulting aqueous solution of a crude PNT-sulfonic acid was cooled to remove 4.5 g of the precipitated PNT-sulfone by filtration. Next, 310 g of an aqueous 30 % sodium hydroxide solution and 310 g of water was added to the filtrate for neutralization. After neutralization, this was heated further to distill out PNT along with water, while water of the same amount as that of the distilled water was continuously supplemented to the system. The distilled water was cloudy as containing PNT as suspended therein. After 1200 ml of water was distilled out, this became transparent. Further, 150 ml of water was distilled out, whereupon the unreacted PNT could no more be detected in the aqueous solution of sodium PNT-sulfonate in the reactor.

The solution thus obtained was concentrated and dried to be a powder. The yield of the sodium PNT-sulfonate obtained was 552 g; and the purity of the same was 93 %.

### EXAMPLE 3

A steam-inleting device-combined 2 liter-reaction flask is equipped with a thermostat-combined packed column (inner diameter: 30 mm, height: 1200 mm). The column was packed with tubular glass chips (outer diameter: 4 mm, inner diameter: 2 mm, length: 4 mm) as a packing material. This column was then equipped with a dropping pipe through which a liquid may drop from the top thereof at a constant rate and a device capable of cooling a distilled water with an warm water of 60 °C and cooling and collecting it. This was used as a steam distillation apparatus for removing an unreacted PNT.

The apparatus was heated with introducing 750 ml/h (as water) of a steam to the packed column from the flask, while an aqueous solution of a crude PNT-sulfonic acid (95 °C) as prepared in the same manner as in EXAMPLE 1 was dropped from the top of the column at a dropping rate of 760 ml/h. The distilled liquid was cloudy as containing PNT in the form of suspension therein. The aqueous solution of PNT-sulfonic acid as retained in the reaction flask had no detectable unreacted PNT.

### EXAMPLE 4

The same steam distillation apparatus as that in EXAMPLE 3 was used, except that the packing material was varied to McMahon Packing (outer diameter: 6 mm). 750 ml/h (as water) of a steam was blown into the packed column from the flask with heating, while 1750 ml/h of an aqueous solution of about 25 % of sodium PNT-sulfonate (kept at 95 °C) was dropped into the column from the dropping pipe at the top thereof. The aqeuous solution was one as prepared in the same manner as in EXAMPLE 2, to which water was added. The distilled liquid was cloudy as containing a suspension of PNT. The aqueous solution of sodium PNT-sulfonate as retained in the bottom of the flask had no detectable unreacted PNT.

### EXAMPLE 5

4.11 kg (30 mols) of a starting material ONT and 0.14 kg of ammonium bicarbonate as an ONT-sulfone producing inhibitor were charged into a 10 liter-reactor (SUS304) equipped with a stirrer, a baffle and a gas-inlet tube. 1.6 m³/h of a dry air was introduced thereinto from the gas-inlet tube for 5 minutes so that ONT in the tube was purged out. Next, 1.6 m³/h of 9 vol.% SO₃ gas was introduced from the tube to initiate sulfonation. The temperature was gradually elevated from room temperature up to from 50 °C to 55 °C to conduct the reaction for 2 hours. Then, the reaction was further conducted at a temperature of 80 °C to 85 °C for 2 hours. Introduction of SO₃ gas (108 mol% of ONT) was stopped in 5 hours including the time for temperature elevation.

The unreacted ONT at the point was 13.6 %, as measured by HPLC analysis. The reaction system was heated further to 110 °C to 120 °C and ripened for 4 hours at the same temperature, whereby the amount of the unreacted ONT decreased to 0.66 %.

For analysis, the product was hydrolyzed, and the sulfuric acid formed was analyzed by potentiometric titration analysis to be 5.50 %. The purity of the thus obtained ONT-sulfonic acid was 92.6 %.

500 g of the ripened product (ONT content: 2.0 wt.%) was dissolved in 600 g of water and heated at 80 °C for 30 minutes to give an aqueous solution of a crude ONT-sulfonic acid. The solution was heated further so as to distill out ONT by steam distillation, while water of the same amount as that of the distilled water was continuously supplemented to the system. The distilled water was cloudy as containing ONT as suspended therein. After 730 ml of water was distilled out, the distilled water became transparent. Further, 80 ml of water was distilled out, whereupon the unreacted ONT could no more be detected in the aqueous solution of ONT-sulfonic acid in the container. The solution was cooled and 5.5 g of ONT-sulfone as precipitated out was removed by filtration. Thus, 1100 g of 42 % aqueous solution of ONT-sulfonic acid was obtained.

### EXAMPLE 6

68.75 g (0.5 mol) of ONT and 2.37 g of ammonium bicarbonate were charged into a 200 ml four-neck flask equipped with a stirrer and a dropping funnel.

42.0 g (105 mol%) of a liquid SO₃ was put in the dropping funnel, and dropping of the liquid to the flask was started.

The temperature and charging of SO₃ were almost same as those in EXAMPLE 5. The dropping time of SO₃ was about 5 hours. At the point, ONT was measured to be 17.5 % by HPLC analysis.

The reaction system was further heated at from 110 °C to 120 °C for 4 hours for ripening, whereupon the unreacted ONT decreased to 1.82 %. The product was hydrolyzed for analysis, and the sulfuric acid formed was determined by potentiometric titration to be 7.4 %. The purity of the ONT-sulfonic acid thus obtained was 91.0 %.

500 g of the ripened product (ONT content: 2.0 wt.%) was dissolved in 600 g of water and heated at 80 °C for 30 minutes to give an aqeuous solution of a crude ONT-sulfonic acid. The solution was cooled to remove 5.5 g of ONT-sulfone as precipitated out, and then this was neutralized with 213 g of 45 % sodium hydroxide. After neutralization, this was heated so as to distill out ONT along with water, while water of the same amount as that of the distilled water was continuously supplemented to the system. The distilled water was cloudy as containing ONT as suspended therein. After 600 ml of water was distilled out, the distilled water became transparent. Further, 70 ml of water was distilled out, whereupon the unreacted ONT could no more be detected in the aqueous solution of sodium ONT-sulfonate in the container. The solution thus obtained was concentrated further and cooled to be a powder. The yield of sodium ONT-sulfonate was 559 g and the purity thereof was 91 %.

### EXAMPLE 7

A steam-introducing device-combined 2 liter-reaction flask was equipped with a thermostat-combined packed column (inner diameter: 30 mm, height: 1200 mm). The colum was packed with tubular glass chips (outer diameter: 4 mm, inner diameter: 2 mm, length: 4 mm) as a packing material. This column was then equipped with a dropping pipe through which a liquid may drop from the top thereof at a constant rate and a device capable of cooling and collecting a distilled water. This was used as a steam distillation apparatus for removing an unreacted ONT.

The apparatus was heated with introducing 610 ml/h (as water) of a steam to the packed column from the flask, while an aqueous solution of a crude ONT-sulfonic acid (as heated to from 80 °C to 90 °C) as prepared in the same manner as in EXAMPLE 6 was dropped from the top of the column at a dropping rate of 3300 ml/h. The distilled liquid was cloudly as containing ONT in the form of a suspension therein. The aqueous solution of ONT-sulfonic acid as retained in the reaction flask had no detectable unreacted ONT.

### EXAMPLE 8

The same steam distillation apparatus as that in EXAMPLE 7 was used, except that the packing material was varied to McMahon Packing (outer diameter: 6 mm). 610 ml/h of a steam was blown into the packed column from the flask with heating, while 4600 ml/h of an aqueous solution of a crude sodium ONT-sulfonate (as heated at 70 °C to 80 °C) as prepared in the same manner as in EXAMPLE 6 was dropped into the column from the dropping tube at the top thereof. The liquid as distilled out by azeotropic distillation was cloudy as containing a suspension of ONT. The aqueous solution of sodium ONT-sulfonate as retained in the bottom of the flask had no detectable unreacted ONT.

The reaction product as obtained by sulfonating a nitrotoluene with SO₃ gas in accordance with the present invention contains a reduced amount of the remaining nitrotoluene and SO₃ due to the interaction of them by ripening of the reaction product. The ripened product is dissolved in water to give an aqueous solution thereof (for sodium salts, the product is neutralized), and the solution may be subjected to steam distillation to remove the unreacted nitrotoluene. As a result, a nitrotoluenesulfonic acid or a sodium nitrotoluenesulfonate which is free from the odor of the nitrotoluene and which has a reduced and controlled amount of the remaining sulfuric acid (for sodium salts, Glauber's salt) can be obtained. The method of the present invention yields no waste acid, though generation of a waste acid was difficult in conventional methods. Therefore, the method of the present invention is free from problems of environmental pollution. The unreacted nitrotoluene as distilled out by steam distillation may be used again as a starting material.

## Claims

1. A method of preparing 2-methyl-5-nitrobenzenesulfonic acid comprising the steps of
reacting p-nitrotoluene with SO₃ gas having an SO₃ concentration of from 3 to 50 vol.%, using from 90 to 130 mol% of SO₃ to p-nitrotoluene (step 1),
ripening the reaction mixture to obtain a reaction product (step 2), and
removing an unreacted p-nitrotoluene from the reaction product (step 3).

2. A method as claimed in claim 1, wherein the step 3 is such that the reaction product is dissolved in water and the solution is subjected to steam distillation to remove an unreacted p-nitrotoluene therefrom.

3. A method as claimed in claim 2, wherein the steam distillation is such that the solution is charged from the top of a packed column while a steam is blown thereinto from the bottom of the column.

4. A method as claimed in claim 1, wherein the reaction product as obtained in the step 2 is dissolved in water to give an aqueous solution of from 30 to 45 %, this is cooled to 40 °C or lower and PNT-sulfone as precipitated out is removed, or the reaction produt is dissolved in water and subjected to steam distillation and then cooled to remove PNT-sulfone.

5. A method of preparing sodium 2-methyl-5-nitrobenzenesulfonate comprising the steps of
reacting p-nitrotoluene with SO₃ gas having an SO₃ concentration of from 3 to 50 vol.%, using from 90 to 130 mol% of SO₃ to p-nitrotoluene (step 1),
ripening the reaction mixture to obtain a reaction product (step 2),
neutralizing the reaction product with sodium hydroxide to obtain a mixture of sodium salt of the reaction product (step 3), and
removing an unreacted p-nitrotoluene from the sodium salt mixture (step 4).

6. A method as claimed in claim 5, wherein the step 4 is such that the mixture is dissolved in water to obtain a solution of the mixture and then this is subjected to steam distillation.

7. A method as claimed in claim 6, wherein the steam distillation is such that the mixture solution is charged from the top of a packed column while a steam is blown thereinto from the bottom of the column.

8. A method as claimed in claim 5, wherein the reaction product as obtained in the step 2 is dissolved in water to give an aqueous solution of from 30 to 45 %, this is cooled to 40 °C or lower and PNT-sulfone as precipitated out is removed therefrom.

9. A method of preparing sodium 4-methyl-3-nitrobenzensesulfonic acid or sodium salt thereof as claimed in anyone of claims 1 to 8, in which o-nitrotoluene is used in place of p-nitrotoluene in the step 1 of claim 1 or 5.
